Europäisches Patentamt
European Patent Office
Office européen des brevets

(19)

(11) Publication number: 0 080 162 B1

(12) EUROPEAN PATENT SPECIFICATION

(45) Date of publication of patent specification: 09.07.86

(51) Int. Cl.⁴: C 07 D 499/00, A 61 K 31/43 // C07D205/08, C07F7/18

(21) Application number: 82110612.7

(22) Date of filing: 18.11.82

(54) 2-(Fluoroalkylthio)substituted penems, processes for preparing them, and pharmaceutical compositions containing them.

(30) Priority: 25.11.81 US 324931

(43) Date of publication of application: 01.06.83 Bulletin 83/22

(45) Publication of the grant of the patent: 09.07.86 Bulletin 86/28

(84) Designated Contracting States: AT BE CH DE FR GB IT LI NL SE

(58) References cited:
EP-A-0 002 210
EP-A-0 003 960

The file contains technical information submitted after the application was filed and not included in this specification

(73) Proprietor: SCHERING CORPORATION
2000 Galloping Hill Road
Kenilworth, New Jersey 07033 (US)

(72) Inventor: Girijavallabhan, Vivyoor Moopil
10 Maplewood Drive
Parsippany New Jersey 07054 (US)
Inventor: Mc Combie, Stuart Walter
159 Pleasant Valley Way
West Orange New Jersey 07052 (US)
Inventor: Ganguly, Ashit Kumar
96 Cooper Avenue
Upper Montclair New Jersey 07043 (US)
Inventor: Pinto, Patrick Anthony
232 Randolph Avenue
Mine Hill New Jersey 07801 (US)
Inventor: Versace, Richard William
230 Lakewood Drive
Ringwood New Jersey 07456 (US)

(74) Representative: Ritter, Stephen David et al
Mathys & Squire 10 Fleet Street
London EC4Y 1AY (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to certain 6-(1-hydroxyethyl)-2-(fluoroloweralkylthio)-penem-carboxylic acids, to pharmaceutically acceptable salts thereof, to metabolisable esters thereof and to pharmaceutical compositions containing them.

The compounds of the present invention have the following general formula:

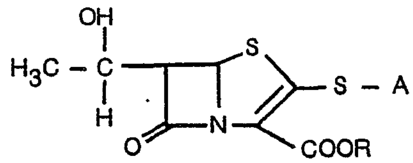

I

in which the stereochemistry is 5R,6S,8R or 5R,6R,8S, wherein A represents a fluoroalkyl group having 2 to 4 carbon atoms, straight chain or branched chain, with 1 to 3 fluorine atoms on a single carbon atom which is separated from the 2—S atom by at least one carbon atom and R is hydrogen, a pharmaceutically acceptable cation or a metabolisable ester group.

The preferred stereochemistry is 5R,6S,8R. The preferred meaning of R is sodium or potassium.

The straight chain fluoroalkyl radicals are derivatives of ethyl, n-propyl and n-butyl. Fluoroethyl radicals are preferred. The most preferred compound is thus, sodium or potassium (5R,6S,8R) 2-(2'-fluoroethylthio)-6-(1-hydroxyethyl)-penem-3-carboxylate.

The branched chain fluoroalkyl radicals include derivatives of isopropyl, sec-butyl and tert-butyl. Branching may e.g. be on the carbon atom adjacent the sulfur atom or on the second carbon atom from the sulfur.

Suitable branched chain fluoroalkyl radicals are

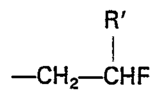

and

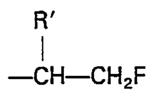

in which R' is methyl or ethyl; and the group

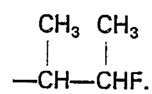

Other typical representatives of A are $CH_2CF_3$, $CH_2CH_2CF_3$ and

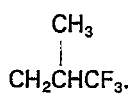

The pharmaceutically acceptable cations referred to above include alkali metal cations such as sodium and potassium, alkaline earth metal cations, e.g. calcium, as well as quaternary ammonium cations for example N-methylglucamine, pyridinium, triethylammonium or triethanolammonium. The sodium and potassium salts are among the preferred embodiments.

The term "metabolisable ester" group denotes a pharmaceutically acceptable ester group which is metabolically removed in the body. Two particularly useful metabolisable ester groups are the phthalidyl group and the pivaloyloxymethyl group.

2-Monofluoroalkylthio compounds of formula I are preparable by fluoridating a compound of the formula

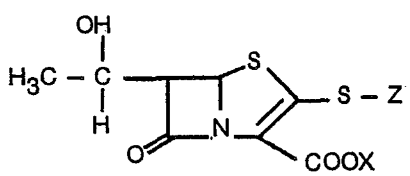

II

in which the stereochemistry corresponds to that of the desired product of formula I, Z is an alkyl group having 2 to 4 carbon atoms, straight chain or branched chain, substituted by a group Lg replaceable by fluorine under the conditions of the reaction, said group Lg being separated from the 2-S atom by at least 2 carbon atoms, X is a carboxy protecting group, and the hydroxy group at position 8 of the penem is protected if required: followed by removal of any protecting group(s) and formation of the free acid, or pharmaceutically acceptable salt, or metabolisable ester.

Lg may, for example, be chosen from hydroxy, bromine, iodine, trifluoro-methyl-sulfonyl, trichloro-methylsulfonyl, arylsulfonyl, and alkylsulfonyl. It is preferably hydroxy.

The fluoridation will generally be carried out by reacting a compound of formula II with a suitable fluoridating agent in a non-hydroxylic, inert solvent. Generally the pH will be about 4 to about 8.

The preferred fluoridating agent is diethylaminosulfur trifluoride (DAST). Other suitable fluoridating agents are e.g. potassium fluoride, $R_4\,^+N\,F^-$ in which R is e.g. an aryl or alkyl radical, sulfur tetrafluoride or the compound

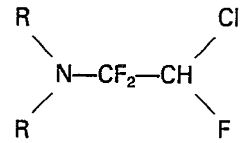

(R as defined above).

Suitable inert solvents are e.g. dichloromethane, trichloromethane, and hexanes.

The fluoridation reaction proceeds adequately at from ambient temperature to −70°C. The preferred temperature range is 25°C. to −20°C.

When the leaving group Lg is not hydroxy, fluoridating agents such as potassium fluoride and $R_4\,^+N\,F^-$ are recommended. In these cases it is preferred that the 8-hydroxy group is not protected.

The requisite intermediates of formula II in which Lg is hydroxy are preferably prepared via stereospecific synthesis which utilizes the procedure described in European Patent Application, Publication No. 0058317. Intermediates of formula II in which Lg is a leaving group other than hydroxy can, if necessary, be prepared from compounds of formula II in which Lg is hydroxy by utilizing conventional esterification techniques and appropriate starting materials. The requisite chiral intermediate, methyl (5R,6S,8R or 5R,6R,8S)-2,2-dimethyl-6-(1-trichloroethoxycarbonyl-oxyethyl)penam-3-carboxylate is prepared via known procedures such as described in the above noted E.P.O. Published Application No. 0013662.

A further process for the preparation of the compounds of formula I comprises alkylating a compound of the formula

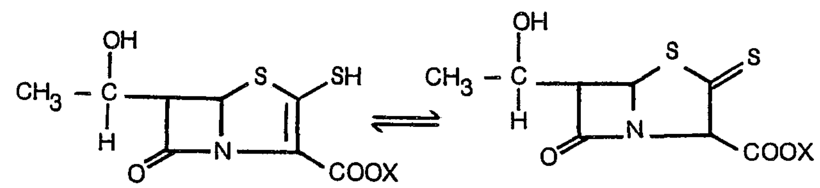

IIIa IIIb

in which the stereochemistry is 5R,6S,8R or 5R,6R,8S, X is a carboxy protecting group and the hydroxy group at the 8-position is protected if desired,
using a compound of the formula

G—Y IV

in which G is an alkyl group having 2 to 4 carbon atoms, straight chain or branched chain, substituted by 1 to 3 fluorine atoms on the same carbon atom which is separated from the atom or group Y by at least one carbon atom, and Y is a group leaving under the conditions of the reaction, followed by removal of any protecting group or groups and isolation of the penem reaction product as a free acid, or pharmaceutically acceptable salt or metabolisable ester.

The intermediates (IIIa, IIIb) are preparable using the procedure described in Japanese published Patent Specification 74762/1980. This procedure involves the reaction of a compound of formula Va

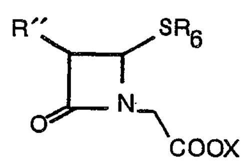

Va

in which R″ is a protected 1-hydroxy-ethyl group, $R_6$ is a lower alkyl group and X is a carboxyl protecting group, with carbon disulfide in the presence of a base such as lithium hexamethyldisilazane followed by reaction with phosgene to produce the compound VI

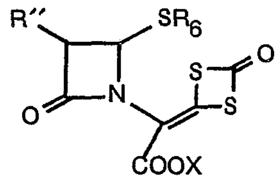

VI

which is then reacted with a halogenating agent such as chlorine or sulfuryl chloride to obtain the compound VII

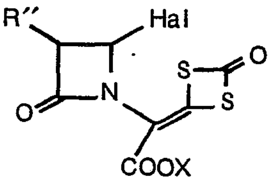

VII

in which Hal is halogen.

Cyclisation of compound VII in the presence of a base such as methylamine or ethylamine yields the tautomeric product

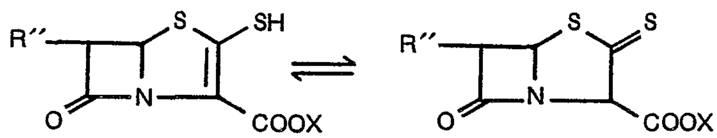

VIIIa VIIIb

Usually, even when starting from stereospecific intermediates, the tautomer product (VIIIa, VIIIb) will be a mixture of diastereoisomers. By using the isomerisation procedure for 5S isomers described in Tetrahedron Letters Vol. 22, page 3485, and/or if necessary conventional separation techniques such as chromatographic separation or fractional crystallisation the substantially pure enantiomers of the required stereochemistry (formula IXa, IXb) can be resolved.

The 8-hydroxy protecting group can be removed, if desired, either before or after resolution of the desired enantiomer.

Alkylation of compound (VIIIa, VIIIb) is usually carried out using a compound IV in which Y is a leaving atom such as chlorine, bromine or iodine, or a leaving group such as alkylsulfonyloxy or arylsulfonyloxy. The reaction will generally be carried out in an inert solvent in the presence of a base, e.g. in tetrahydrofuran or a halogenated hydrocarbon such as chloroform, in the presence of an organic base such as pyridine or an alkali metal carbonate such as potassium carbonate. Suitable alkylating temperatures are between −10°C and 100°C.

Yet a further process for the production of compounds of formula I comprises reacting a compound of the formula V

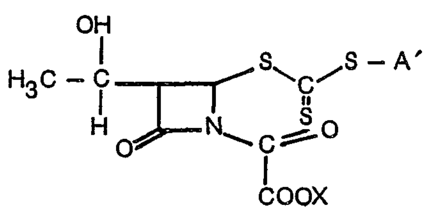

V

in which the stereochemistry in 3S,4R,5R or 3R,4R,5S, A′ is a fluoroalkyl group, straight or branched chain, with 1 to 3 fluorine atoms on the same carbon atom which is separated from the adjacent sulphur atom by at least one carbon atom, X is a carboxy protecting group and, if necessary, the 5-position hydroxy group is protected,

with a trivalent organophosphorus compound e.g. triethylphosphite, or triphenylphosphite;

followed by removal of the protecting group or groups and isolation of the penem reaction product as a free acid, pharmaceutically acceptable salt, or as a metabolisable ester.

The required intermediate of formula V can be prepared by, and the conversion thereof can be analogous to, the procedures set forth in our European Patent Application, Publication No. 0058317.

Thus the intermediate azetidinone of formula V can be prepared by reacting an azetidinone of formula XI.

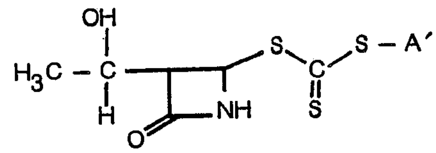

XI

in which A′ is as defined above, with an acid halide of formula

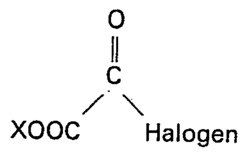

in an inert solvent, e.g. benzene or toluene, in the presence of an alkaline earth metal carbonate or alkali metal carbonate at e.g. 20°C to 80°C.

The carboxy protecting groups X and hydroxy protecting groups used in the above described processes may be any of the conventional protecting groups used in penem chemistry, see for example our European Patent Application publication No. 0013662. The preferred carboxy protecting groups are allylic groups, e.g. 2-chloroallyl, and most preferably allyl. The preferred hydroxy protecting group is trichloroethoxycarbonyl.

Other suitable hydroxy protecting groups are e.g., benzyloxycarbonyl, p-nitrobenzyloxycarbonyl, benzhydryloxycarbonyl, and allyloxycarbonyl.

Other suitable carboxy protecting groups are e.g. benzyl, p-nitrobenzyl and benzhydryl.

Removal of an 8-hydroxy protecting group is common in the penem art. The preferred protecting group (trichloroethoxycarbonyl) can be removed by reaction with zinc in acetic acid.

The carboxy protecting group X can likewise be removed by conventional procedures. The preferred group(s) X are most conveniently removed utilizing, procedures described in our European Patent Application Publication No. 0013663.

Thus, the allylic groups are preferably removed by utilizing a suitable aprotic solvent, such as tetrahydrofuran, diethyl ether or methylene chloride, with potassium or sodium 2-ethylhexanoate or 2-ethylhexanoic acid and a mixture of a palladium compound and a triarylphosphine compound e.g. triphenylphosphine as a catalyst.

When hexanoic acid is used to remove the allylic protecting group, there is formed a compound of formula I wherein R is hydrogen; whereas, when sodium or potassium hexanoate is used, there is formed a compound of formula I wherein R is sodium or potassium, respectively.

Other pharmaceutically acceptable salts of formula 1 may be prepared from the sodium or potassium salts by methods known in the art such as replacement by ion exchange of an aqueous solution of the sodium salts. Similarly, metabolisable esters of formula 1, e.g. the pivaloyloxymethyl and phthalidyl esters may be prepared from the sodium or potassium salts utilizing known procedures.

The compounds of this invention possess antibacterial activity of both the gram-positive and gram-negative type. Most importantly, they are orally active antibacterial agents which afford good blood levels at antibacterial dosages. When tested in standardized microbiological assays, the compounds of this invention are active against such gram-positive organisms as *Staphylococcus epidermidis*, and *Bacillus subtilis* and such gram-negative organisms as *E. coli* and *Salmonella* at test levels of 0.1 to 100 µg/ml. Additionally, they show activity against such organisms which produce beta-lactamases, *e.g.,* penicillanase and cephalosporinase, indicating a resistance against these enzymes. For instance, sodium (5R,6S,8R)-6-(1-hydroxyethyl)-2-(2′-fluoroethylthio)-penem-3 carboxylate and the corresponding potassium salt are active against *Staphylococcus* 7607010 at a test level of 0.5 µg/ml. When tested against *B. subtilis* 1119601 (a beta-lactamase-containing organism), these compounds exhibit activity at 0.06 µg.ml.

The present invention includes within its scope pharmaceutical compositions comprising an antibacterially effective amount of a penem of formula 1 together with a compatible pharmaceutically acceptable carrier or coating. The compounds of formula 1 may be the only antibacterial agent in the pharmaceutical dosage forms, or may be admixed with other compatible antibacterial agents and/or enzyme inhibitors.

Preferred embodiments concern the pharmaceutical compositions suitable for oral administration. A particularly preferred embodiment relates to a pharmaceutical composition which is an oral antibacterial dosage unit comprising an antibacterially effective amount of compound of formula I, especially where R is sodium or potassium, e.g., sodium (5R,6S,8R)-6-(1-hydroxyethyl)-2-(2′-fluoroethylthio)-penem-3-carboxylate, together with a compatible pharmaceutically acceptable carrier. Of these compositions, those which are solid are particularly useful. The oral dosage forms are characterized by an unusual combination of high potency or prolonged duration, broad antibacterial spectrum and efficacy via the oral route of administration.

The dosage administered of the penems of this invention is dependent upon the age and weight of the animal species being treated, the exact mode of administration, and the type and severity of bacterial infection being prevented or reduced. Typically, the dosage administered per day will be in the range of 5 to 200 mg/kg per day with 20 to 80 mg/kg per day being preferred.

For oral administration, the compound of this invention may be formulated in the form of tablets, capsules, elixirs or the like. Likewise, they may be admixed with animal feed. They may also be applied topically in the form of ointments, both hydrophilic and hydrophobic, in the form of lotions which may be aqueous, non-aqueous or of the emulsion type, or in the form of creams.

The compounds of formula I may be utilized in liquid form such as solutions, suspensions, and the like for otic and optic use and may also be administered parenterally via intramuscular injection.

Representative compounds of the present invention are:

1. sodium or potassium (5R,6S,8R)-2-(2′-fluoroethylthio)-6-(1-hydroxyethyl)penem-3-carboxylate;
2. sodium or potassium (5R,6S,8R)-2-(3′-fluoropropylthio)-6-(1-hydroxyethyl)penem-3-carboxylate;
3. sodium or potassium (5R,6S,8R)-2-(2′-fluoropropylthio)-6-(1-hydroxyethyl)penem-3-carboxylate;
4. sodium or potassium (5R,6S,8R)-2-(1′-fluoromethylethylthio)-6-(1-hydroxyethyl)penem-3-carboxylate;
5. sodium or potassium (5R,6S,8R)-2-(1′-fluoromethylpropylthio)-6-(1-hydroxyethyl)penem-3-carboxylate;
6. sodium or potassium (5R,6S,8R)-2-(2′-fluoromethylpropylthio)-6-(1-hydroxyethyl)penem-3-carboxylate;
7. sodium or potassium (5R,6S,8R)-2-(2′,2′,2′-trifluoroethylthio)-6-(1-hydroxyethyl)penem-3-carboxylate;

the free acids and the pivaloyloxymethyl esters and phthalidyl esters thereof, as well as the (5R,6R,8S) isomers of the previously mentioned acids, salts and esters.

## Example 1

A) (3S,4R,5R)-3-(1-trichloroethoxycarbonyloxyethyl)-4-(2-hydroxyethylthiocarbothioylthio)-azetidin-2-one

Dissolve 10 g of methyl-(5R,6S,8R)-2,2-dimethyl-6-(1-trichloroethoxycarbonyloxyethyl)-penam-3-carboxylate in 150 ml of methylene chloride at 0—5°C., add 7.36 ml of sulfuryl chloride and stir for one hour at room temperature. Pour the reaction mixture into an excess of aqueous sodium bicarbonate with stirring. Separate the two liquid phases, dry the organic phase and evaporate to a residue. Dissolve the residue in 100 ml of methylene chloride and treat with ozone at −78°C. until a blue color persists, add 5 ml of dimethyl sulfide to the reaction mixture at room temperature and stir for one hour, then add this mixture to a stirred ice cold trithiocarbonate solution prepared from 10 ml of beta-mercaptoethanol and 6 g of potassium hydroxide in 200 ml of 50% aqueous ethanol cooled to 0°C., and treated with 28 ml of carbon disulfide. Allow the mixture of the chlorolactam and the trithiocarbonate solution to react at 0°C. for 45 minutes with stirring, then dilute with water. Extract the reaction mixture with methylene chloride, wash aqueous sodium bicarbonate, dry over magnesium sulfate and evaporate to a residue. Chromatograph on silica gel, eluting with an increasing concentration of ethyl ether in methylene chloride to 30%. Combine like fractions containing the title compound as determined by thin layer chromatography and evaporate to obtain the product of this step as a light yellow oil.

Yield — 8.1 g.
I.R. ($CH_2Cl_2$) 3550, 1770, 1750 $cm^{-1}$.

B) (3S,4R,5R)-3-(1-trichloroethoxycarbonyloxyethyl)-4-[2-(t-butyldimethylsilyloxy)ethylthiocarbothioylthio]-azetidin-2-one

Dissolve 7.07 g of the product from Step A in a mixture of 50 ml of methylene chloride and 1.43 ml of pyridine, 2.64 g of *t*-butylchlorodimethylsilane and 0.1 g of imidazole. Stir the solution at room temperature for two days, wash with water and evaporate to a residue. Chromatograph the residue on silica gel using dichloromethane:hexane and then methylene chloride with increasing concentrations of ethyl-ether. Combine like fractions containing the title compound as determined by thin layer chromatography and evaporate to obtain the title compound as a light yellow oil.

Yield — 8.4 g.
I.R. 3400, 1700 and 1750 $cm^{-1}$.

C) Allyl-(5R,6S,8R)-2-[2-(t-butyldimethylsilyloxy)ethylthio]-6-(1-trichloroethoxycarbonyloxyethyl)-penem-3-carboxylate

Dissolve 8.44 g of the product of Step B in 50 ml of methylene chloride containing 2.69 g of allyloxalylchloride and stir at 0—5°C. while adding 2.32 g of diisopropylethylamine in 15 ml of methylene chloride dropwise. Stir the reaction mixture for an additional half-hour at 0—5°C., wash with water, then with dilute hydrochloric acid and then with dilute aqueous sodium bicarbonate. Dry the organic solvent phase over magnesium sulfate, filter and evaporate to a residue. Dissolve the residue in 100 ml of ethanol-free chloroform, add 1.0 g of calcium carbonate and reflux with stirring during the addition of 5 g of triethyl phosphite over a 3-hour interval. Reflux the solution for an additional 18 hours, cool and chromatograph on silica gel eluting with methylene chloride:hexane, methylene chloride and finally with 1% ethyl ether in

methylene chloride. Combine like fractions containing the title compound as determined by thin layer chromatography to obtain thereby the title compound as a yellowish oil.

'H NMR ($CDCl_3$): 0.10 (s, 6), 0.92 (s, 9), 1.54 (d, 3, J = 7), 3.07 (n, 2), 3.84 (m, 3), 4.76 (m, 2), 4.79 (s, 2), 5.1—5.6 (m, 3), 5.64 (d, 1, J = 2.5) and 5.7—6.2 (m, 1).

D) Allyl-(5R,6S,8R)-2-(2-hydroxyethylthio)-6-(1-trichloroethoxycarbonyloxyethyl)-penem-3-carboxylate

Dissolve 4.46 g of the product of Step C in a mixture of 32 ml of tetrahydrofuran, 4 ml of water and 4 ml of acetic acid. Stir the solution for 18 hours at room temperature with 2.4 g of tetra-n-butylammonium fluoride. Pour the reaction mixture into a two-phase solvent system consisting of methylene chloride and water with stirring. Wash the organic phase with aqueous sodium bicarbonate. Dry the organic phase over magnesium sulfate, filter and evaporate to a residue. Chromatograph the residue on silica gel using ethyl ether:methylene chloride as the eluant. Combine like fractions containing the title compound as determined by thin layer chromatography and evaporate to obtain thereby the title compound of this example as a yellowish oil.

Yield — 2.9 g.

'H NMR ($CDCl_3$): 1.49 (d, 3, J = 7), 2.17 (m, 1, exch by $D_2O$), 3.12 (m, Z), 3.70—1.0 (m, 3), 4.72 (m, 2), 4.76 (s, 2), 5.1—5.6 (m, 3), 5.67 (d, 2, J = 2.5) and 5.7—6.2 (m, 1).

E) Allyl(5R,6S,8R)-2-(2-fluoroethylthio)-6-(1-trichloroethoxycarbonyloxyethyl)-penem-3-carboxylate

Dissolve 400 mg of the product of Step D and suspend 1 g of calcium carbonate in 25 ml of methylene chloride at −78°C. Add 0.35 ml of diethylaminosulfur trifluoride (DAST) and stir for ½ hour. Dilute with ethyl acetate. Stir with water for five minutes at 0°C. Separate layers and wash the organic solvent layer with water. Concentrate the organic layer to a residue and chromatograph on silica gel using ethyl ether:methylene chloride as the eluant. Combine the fractions containing like products as determined by thin layer chromatography and evaporate to obtain the title product.

Yield 200 mg.

NMR 6.2—5.8 (1H, m); 5.7 (1H, d, J = 1 cps); 5.5—5.3 (3H); 4.85 (1H, J = 6 cps and 50 cps); (F—CH coupling); 3.95 (1H, d, d, J = 1 cps) — 9 cps; 3.25 [tt (S—$CH_2$)]; 1.4 (3H, d, J = 9 cps).

I.R. 1795 $cm^{-1}$, 1765 $cm^{-1}$, 1695 $cm^{-1}$.

F) Allyl(5R,6S,8R)-2-(2-fluoroethylthio)-6-(1-hydroxyethyl)-penem-3-carboxylate

Dissolve 200 mg of the product of Step E in 10 ml of tetrahydrofuran. Add 300 mg of zinc, 0.5 ml of acetic acid and 0.5 ml of water. Stir the mixture at −5° to 0°C. for 1½ hours. Extract with ethyl acetate, wash the extract with aqueous calcium carbonate solution and filter. Concentrate the ethyl acetate solution to a residue and chromatograph on a silica gel column using 10% ethyl acetate solution to a residue and chromatograph on a silica gel column using 10% ethyl acetate/methylene chloride as the eluant. Combine the fractions containing like products as determined by thin layer chromatography. Evaporate the combined fractions to obtain thereby the title product.

Yield 100 mg.

NMR 6.2—5.8 (1H, m); 5.7 (1H, d, J = 1.5 cps); 5.5—5.3 (2H); 1H, J = 7 and 50 cps); 44.35 (1Ht, J = 50 cps); 3.75 (1H, dd, J = 1.5 and 9 cps); 3.3 (2H, m); 1.4 (3H, d, J = 9 cps).

I.R. 3400 $cm^{-1}$, 1795 $cm^{-1}$, 1720 $cm^{-1}$ and 1695 $cm^{-1}$.

G) Sodium(5R,6S,8R)-2-(2-fluoroethylthio)-6-(1-hydroxyethyl)-penem-3-carboxylate

Dissolve 86 mg of the product of Step F in 10 ml of methylene chloride. Add 48 mg of sodium hexanoate, 28 mgs of tetrakis (triphenylphosphine) palladium and 30 mg of triphenylphosphine. Stir the reaction mixture under nitrogen for ½ hour. Extract the methylene chloride with water. Separate the layers. Extract the organic layer with ethyl acetate, wash with water, treat the water layer with 8 mg of sodium bicarbonate and add the aqueous layer to the previously obtained aqueous layer and lyophilize. Dissolve the so-obtained penem salt in 3.0 ml of water and, using pressure, force the aqueous phase through $C_{18}$ silica gel followed by a water wash. Lyophilize the aqueous column eluate and wash to obtain thereby the title compound.

Yield 30 mgs.

$(\alpha)_D^{26} = +150.8°$ [$H_2O$].

NMR, $D_2O$ 5.75, 1H, d (J = 1Hz); 5.05 and 4.4 (2 triplets —$CH_2$—F 2H, (J = 1.8 Hz, 9 Hz); 4.3, 1H, m; 3.95, 1H, dd, J = 1 and Hz; 3.3, 2H m; 2.2, 1H (exchangable — O<u>H</u>); 1.3, 3H, d, J = 9 Hz.

In a similar manner, when sodium hexanoate of Step G, Example 1 is replaced by an equivalent quantity of hexanoic acid the corresponding penem carboxylic acid is produced i.e. (5R,6S,8R)-2-(2′-fluoroethylthio)-6-(1-hydroxyethyl)-penem-3-carboxylic acid.

Other pharmaceutically acceptable alkali metal salts (e.g. potassium) may be prepared from the sodium salt by methods generally known in the art, such as replacement by ion exchange of an aqueous solution of the sodium salt or by treating a penem carboxylic acid with base having the desired cation. In either event, the pharmaceutically acceptable salt is preferably isolated by lyophilization. The corresponding phthalidyl and pivaloyloxymethyl metabolisable esters can be prepared by analogy to the procedure described in Example 2 of European Patent Application No. 0013662, e.g. by reaction of the sodium or potassium salts with phthalidyl chloride or pivaloyloxymethyl chloride, in dry tetrahydrofuran in the presence of sodium iodide.

In like manner, starting from the appropriate intermediates the following compounds can be prepared:

sodium or potassium (5R,6S,8R)-2-(3′-fluoropropylthio)-6-(1-hydroxyethyl)penem-3-carboxylate;

sodium or potassium (5R,6S,8R)-2-(2′-fluoropropylthio)-6-(1-hydroxyethyl)penem-3-carboxylate;

sodium or potassium (5R,6S,8R)-2-(1′-fluoromethylethylthio)-6-(1-hydroxyethyl)penem-3-carboxylate;

sodium or potassium (5R,6S,8R)-2-(1′-fluoromethylpropylthio)-6-(1-hydroxyethyl)penem-3-carboxylate;

sodium or potassium (5R,6S,8R)-2-(2′-fluoromethylpropylthio)-6-(1-hydroxyethyl)penem-3-carboxylate;

as well as the corresponding free acids and phthalidyl and pivaloyloxy methyl esters.

As previously stated, the compounds of this invention are antibacterially effective against strains of both Gram (+) and Gram (−) bacteria including anaerobes. Examples 2 through 8 which follow are directed to some of the dosage forms which may be employed to administer the compounds of this invention. In the formulations the word "Drug" means sodium or potassium salts of (5R,6S,8R)-6-(1-hydroxyethyl)-2-(2′-fluoroethylthio)-penem-3-carboxylic acid or (5R,6S,8R)-6-(1-hydroxyethyl)-2-(3′-fluoro-n-propylthio)-3-carboxylic acid or an equivalent amount of the free 3-carboxylic acid compound or the phthalidyl or pivaloyloxymethyl esters thereof.

It will be appreciated, however, that these compounds may be replaced by an equally effective quantity of another compound defined by formula I, especially a compound comprised in the list and passage subsequent thereto immediately preceding the Examples.

### Example 2

*Injection Formulation*

Per vial: Drug (Sterile powder).

Exemplary unit dosages may be 125 mg., 250 mg., 500 mg., 1 gm. and 2 gms. Add sterile water for injection U.S.P. or bacteriostatic water for injection U.S.P., for reconstitution.

### Example 3

*Capsule Formulation*

| Item No. | Ingredient | mg/capsule | mg/capsule |
|---|---|---|---|
| 1 | Drug | 250 | 500 |
| 2 | Microcrystalline Cellulose | 30 | 60 |
| 3 | Corn Starch, Dried | 15 | 30 |
| 4 | Silica Gel | 4.5 | 9 |
| 5 | Magnesium Stearate | 0.5 | 1 |
| | | 300.0 mg | 600 mg |

*Method*

Mix Item Nos. 1, 2, 3 and 4 in a suitable mixer for 10—15 minutes. Add Item No. 5 and mix for 1—3 minutes. Fill the above mixture in two-piece hard gelatin capsules of required size.

Alternatively, mix Item Nos. 1, 2, 3 and 4 in a suitable mixture for 10—15 minutes. Add half the amount of Item No. 5, mix for 1—3 minutes. Pass the mixture through a suitable compactor. Pass the impacted mixture through a suitable mill equipped with 16 mesh screen. Remix and add the remainder amount of Item No. 5. Mix for 1—3 minutes. Fill the above mixture in two-piece hard gelatin capsules of required size.

Example 4

*Tablet Formulation*

| Item No. | Ingredient | mg/tablet | mg/tablet |
|---|---|---|---|
| 1 | Drug | 250 | 500 |
| 2 | Microcrystalline Cellulose | 100 | 200 |
| 3 | Corn Starch, Dried | 40 | 80 |
| 4 | Silica Gel | 6 | 12 |
| 5 | Magnesium Stearate | 4 | 8 |
| | | 400 mg | 800 mg |

*Method*

Mix Item Nos. 1, 3 and half the amount of Item No. 4 in a suitable mixer for 10—15 minutes. Add half the amount of Item No. 5 and mix for 1—3 minutes. Pass the mixture through a suitable compactor. (Alternatively, slug the mixture on a rotary tablet machine equipped with 1″ flat bevelled punches). Mill the compacted material or the slugs using a suitable milling machine equipped with 16 mesh screen. Remix. Add Item No. 2 and the remainder amount of Item No. 4. Mix for 10—15 minutes. Add the balance of Item No. 5 and mix for 1—3 minutes. Compress the mixture into the tablets of required shape and size on a rotary tablet machine. The tablets may be coated using standard coating procedures.

Example 5

*Topical Formulation*

| Item No. | Ingredient | mg/g |
|---|---|---|
| 1 | Drug | 25 |
| 2 | Ethyl Alcohol | 400 |
| 3 | Hydroxypropyl Cellulose | 15 |
| 4 | Polyethylene Glycol 400 | 560 |

Mix Item Nos. 1, 2, and 4 in a suitable mixer. Stir vigorously and charge Item No. 3. Maintain stirring until uniformity is achieved.

Example 6

*Oral Powder for Reconstitution (I)*

Part A (Powder Formulation)

| Item No. | Ingredient | mg/g |
|---|---|---|
| 1 | Drug | 46.3 |
| 2 | Flavour(s) | q.s. |
| 3 | Colorant | q.s. |
| 4 | Preservative | q.s. |
| 5 | Buffering Agents | q.s. |
| 6 | Saccharin | 28.3 |
| | To make | 1.0 g |

Mix Item Nos. 1, 2, 3, 4 and 5 thoroughly, Charge Item No. 6 and mix until uniformity is achieved.

Part B (Reconstitution)

Charge 54 g of above formulated powder into a proper container and add enough water to make up 100 ml. Shake well after the addition of water. Each 5 ml (1 teaspoonful) will then contain drug equivalent to 125 mg.

Example 7

*Oral Liquid*

| Item No. | Ingredient | mg/ml |
|---|---|---|
| 1 | Drug | 25.0 |
| 2 | Sweetener | q.s. |
| 3 | Flavor | q.s. |
| 4 | Colorant | q.s. |
| 5 | Vegetable Oil | q.s. |
| | To make | 1.0 ml |

Charge 90% of Item No. 5 needed into a suitable container. Charge Item Nos. 1, 2, 3 and 4 and mix well. Bring to the final volume by the reserved Item No. 5.

Example 8

*Suppository*

| Item No. | Ingredient | parts by weight |
|---|---|---|
| 1 | Drug | 125.0 |
| 2 | Witepsol H—15 | 1868 |

Melt Item No. 2 and blend Item No. 1 until uniform. Pour into mold and congeal in refrigerator. Remove suppository from mold.

Example 9

Sodium (5R,6S,8R)-6-(1-hydroxyethyl)-2-(2′-fluoroethylthio)penem-3-carboxylate

A. To a solution of (3S,4R,5R)-3-(1-2′,2′,2′trichloroethoxycarbonyloxyethyl)-4-(2″-fluoroethylthio)-carbonothioylthio)-azetidin-2-one (0.654 g) in 6 ml. methylenechloride cooled to 10°C, add with stirring, 0.6 g calcium carbonate followed by 0.263 g (1.2 equiv.) allyloxyoxallylchloride. Then add dropwise a solution of diisopropylethylamine (0.32 ml., 1.2 equiv.) in 1 ml. methylene chloride over 5 minutes maintaining the temperature in the range 10°C.

When, after 15 minutes, thin layer chromatography indicates no starting compound (the temperature being 15°C) transfer the mixture to a separating funnel using ethanol-free chloroform. Wash the resulting mixture twice with ice/water, filter to remove excess calcium carbonate, dry over anhydrous sodium sulfate and transfer to a 100 ml. 3-neck flask. Adjust the volume of the solution to approximately 50 ml. with chloroform and reflux while adding a solution of triethylphosphite (0.6 ml., 2 equiv.) in 20 ml. chloroform over 3 hours. Reflux the mixture for an additional 18 hours, evaporate and chromatograph on 14 g silica gel, eluting with 25 per cent ether-hexane. Combine and evaporate like eluates to obtain a residue (420 mg) comprising allyl(5R,6S,8R)-6-(1-trichloroethoxycarbonyloxyethyl)-2-(2′-fluoroethylthio)-2-penem-3-carboxylate (58% yield). Purify by crystallisation from an ether-hexane mixture to obtain the aforesaid allyl ester in crystalline form in a yield of 330 mg (46% theoretical).

B. Treat the product from step A according to the procedures set forth in step F and then step G of Example 1 to obtain the title compound.

In like manner, starting from the appropriate intermediates the following compounds can be prepared:

sodium or potassium (5R,6S,8R)-2-(3′-fluoropropylthio)-6-(1-hydroxyethyl)penem-3-carboxylate;

sodium or potassium (5R,6S,8R)-2-(2′-fluoropropylthio)-6-(1-hydroxyethyl)penem-3-carboxylate;

sodium or potassium (5R,6S,8R)-2-(1′-fluoromethylethylthio)-6-(1-hydroxyethyl)penem-3-carboxylate;

sodium or potassium (5R,6S,8R)-2-(1′-fluoromethylpropylthio)-6-(1-hydroxyethyl)penem-3-carboxylate;

sodium or potassium (5R,6S,8R)-2-(2′-fluoromethylpropylthio)-6-(1-hydroxyethyl)penem-3-carboxylate;

sodium or potassium (5R,6S,8R)-2-(2′2′2′-trifluoroethylthio)-6-(1-hydroxyethyl)penem-3-carboxylate; and the corresponding free acids and phthalidyl and pivaloyloxy methyl esters, can be obtained by following the procedures mentioned in the last part of Example 1.

Example 10

A. Dissolve approximately 250 mg of an equilibrium mixture of allyl (5R,6S,8R)-6-(1-hydroxyethyl)-2-thione penam-3-carboxylate and allyl (5R,6S,8R)-6-(1-hydroxyethyl)-2-thiol-penem-3-carboxylate in 3 ml. tetrahydrofuran. Add 1.5 equivalents of 2,2,2-trifluoroethyl-trifluoromethane sulfonate ($CF_3SO_2CH_2CF_3$). Then add 1 equivalent of potassium carbonate in powder form to the reaction mixture and stir for 2 hours at room temperature. Filter the reaction mixture and then wash with a 2% solution of phosphoric acid in methylene chloride. Evaporate the methylene chloride solution and obtain a semi-crystalline residue. Dissolve this residue in a warm 50:50 mixture of trichloromethane and petroleum ether, cool and collect a first crop of white crystals. Cool further and collect a second crop of white crystals. Treat the mother liquor on a silica gel column eluting with 95% methylene chloride/5% ethyl acetate. Collect like fractions and evaporate the solvent to give a total yield of 160 mg of pure allyl (5R,6S,8R)-6-(1-hydroxyethyl)-2-(2′,2′,2′-trifluoroethylthio) penem-3-carboxylate.

B. Dissolve the 160 mg of allyl ester from step A. in 2 ml. methylene chloride. To the resulting solution add approximately 50 mg triphenylphosphite, then 4 equivalents of 1 m pyridinium formate containing 10% excess pyridine. To the reaction mixture add in several portions, 250 ml. of a saturated solution of (triphenyl) phosphine palladium —(O) in methylene chloride. Run the reaction for 3 hours by which time it is complete. Wash the reaction mixture with a 2% aqueous solution of phosphoric acid.

Extract the phosphoric acid solution (pH≈1) four times with ethyl acetate and evaporate to obtain 59 mg of (5R,6S,8R)-6-(1-hydroxyethyl)-2-(2′,2′,2′-trifluoroethylthio) penem-3-carboxylic acid.

C. Dissolve the free acid in 5 ml. of a 50:50 mixture of tetrahydrofuran and water, add 1 equivalent of sodium bicarbonate also in a 50:50 mixture of tetrahydrofuran and water and lyophilize to give (5R,6S,8R)-6-(1-hydroxyethyl)-2-(2′,2′,2′-trifluoroethylthio) penem-3-carboxylate.

In like manner, starting from the appropriate intermediates the following compounds can be prepared:

Sodium or potassium (5R,6S,8R)-2-(3′-fluoropropylthio)-6-(1-hydroxyethyl)penem-3-carboxylate;

Sodium or potassium (5R,6S,8R)-2-(2′-fluoropropylthio)-6-(1-hydroxyethyl)penem-3-carboxylate;

Sodium or potassium (5R,6S,8R)-2-(1′-fluoromethylethylthio)-6-(1-hydroxyethyl)penem-3-carboxylate;

Sodium or potassium (5R,6S,8R)-2-(1′-fluoromethylpropylthio)-6-(1-hydroxyethyl)penem-3-carboxylate;

Sodium or potassium (5R,6S,8R)-2-(2′-fluoromethylpropylthio)-6-(1-hydroxyethyl)penem-3-carboxylate;

Sodium or potassium (5R,6S,8R)-2-(2-fluoroethylthio)-6-(1-hydroxyethyl)penem-3-carboxylate;

as well as the corresponding free acids and phthalidyl and pivaloyloxy methyl esters.

Antibacterial tests on Müller-Hinton Agar at pH 7 with compound 1 indicated minimum inhibitory concentrations (µg/ml) required to inhibit growth of the following bacteria of the strain collection of Schering Corporation to be as given in Table 2.

TABLE 2

| Organism | MIC | Organism | MIC |
|---|---|---|---|
| *Acinetobacter* | | *E.coli* | |
| 77080902 | 0.125 | 79121101 | 0.1250 |
| 79121104 | 2.000 | 75020706 | 0.5000 |
| 77080901 | 0.5000 | 001574—1 | 0.2500 |
| 77080954 | 2.0000 | 79101902 | 0.2500 |
| 75091623 | 1.0000 | 80091808 | 1.0000 |
| 77041312 | 1.0000 | 79121109 | 0.1250 |
| 79062004 | 0.2500 | 80091813 | 0.2500 |
| 78120401 | 0.5000 | 79012503 | 0.2500 |
| 80070723 | 0.1250 | 80091812 | 0.5000 |
| 77080952 | 1.0000 | 79043017 | 0.1250 |
| 77062909 | 2.0000 | 80080408 | 0.2500 |
| 76042103 | 0.1250 | 79111505 | 0.1250 |
| *Citrobacter* | | 75082817 | 0.1250 |
| 76022001 | 0.2500 | 80103108 | 0.2500 |
| 79012521 | 0.1250 | 80100203 | 1.0000 |
| 79012543 | 0.1250 | 77081847 | 0.2500 |
| 79121126 | 0.2500 | *Klebsiella* | |
| 75041104 | 0.2500 | 76040112 | 0.1250 |
| 77062904 | 0.1250 | 76031502 | 0.1250 |
| 80080412 | 0.1250 | 78100504 | 0.2500 |
| 80080445 | 0.2500 | 80020801 | 0.1250 |
| 77080906 | 0.2500 | 74041603 | 0.2500 |
| 79040613 | 0.1250 | 75032102 | 0.1250 |
| 79112613 | 0.5000 | 79111503 | 0.2500 |
| 79030103 | 0.2500 | 80020809 | 0.2500 |
| 80103101 | 0.5000 | 80102909 | 0.2500 |
| 76050403 | 0.2500 | 79100201 | 0.1250 |
| 76041201 | 0.2500 | 76082701 | 0.2500 |
| 77081846 | 0.2500 | 80090882 | 1.0000 |

TABLE 2 (continued)

| Organism | MIC | Organism | MIC |
|---|---|---|---|
| 75041106 | 0.2500 | 80103112 | 0.2500 |
| 77080949 | 0.2500 | 80103115 | 0.2500 |
| 80102913 | 0.2500 | 74120401 | 0.2500 |
| 77080903 | 0.5000 | 80020810 | 0.2500 |
| *Enterobacter* | | *Morganella* | |
| 79101907 | 0.2500 | 75091615A | 1.0000 |
| 72012502 | 0.5000 | 77020408 | 1.0000 |
| 76052901 | 0.1250 | 77081842 | 0.5000 |
| 77081832 | 0.2500 | 80082106 | 1.0000 |
| 77081804A | 0.2500 | 80090859 | 0.5000 |
| 79062510 | 0.2500 | 75051303 | 0.5000 |
| 79040605 | 0.1250 | 75082809 | 1.0000 |
| 75043005 | 0.2500 | 77080933 | 1.0000 |
| 79070501 | 1.0000 | 77080932 | 2.0000 |
| 80103104 | 0.2500 | 80090864 | 0.5000 |
| 72012503 | 2.0000 | 80103157 | 1.0000 |
| 79030901 | 1.0000 | 72012507 | 1.0000 |
| 75043003 | 0.2500 | 76120901 | 1.0000 |
| 78111402 | 0.5000 | 79040664 | 2.0000 |
| 77080959 | 0.5000 | 80091806 | 1.0000 |
| 77061306 | 0.2500 | *Prov. stuartii Salmonella-Shigella* | |
| 77081802 | 0.5000 | 74111801A | 0.5000 |
| 77080934 | 0.5000 | OC2 NEWPT | 0.1250 |
| 77062906 | 0.5000 | 76061713 | 0.1250 |
| 74050202 | 0.5000 | 00013313 | 0.2500 |
| 80032105 | 0.5000 | OGRGCUBA | 0.2500 |
| 80080423 | 0.2500 | 77072001 | 0.2500 |
| 77061304 | 1.0000 | 79112610 | 0.2500 |
| 80081203A | 0.1250 | 77072002 | 0.5000 |
| 72012501 | 4.0000 | 76061707 | 0.2500 |
| 78022362 | 0.2500 | 76061716 | 0.2500 |

TABLE 2 (continued)

| Organism | MIC | Organism | MIC |
|---|---|---|---|
| 77081828 | 1.0000 | 74060311 | 0.2500 |
| 77081828 | 1.0000 | 79103101 | 0.5000 |
| 75081106 | 1.0000 | 77053109 | 0.1250 |
| 80060602 | 0.5000 | 80082103 | 0.2500 |
| 80090860 | 2.0000 | 76061701 | 0.2500 |
| 80092501 | 1.0000 | 80081402 | 0.2500 |
| *Proteus/ Providencia* | | 78101707 | 0.2500 |
| | | *Staphylococcus* | |
| 770020403 | .2500 | 00000868 | 0.0625 |
| 77020404 | .2500 | 76070107 | 0.2500 |
| 77020402 | .2500 | 000SG511 | 0.0625 |
| 75082803 | .5000 | 78011916 | 0.2500 |
| 78071009 | .5000 | 00000211 | 0.0625 |
| 77080960 | .2500 | 78053002 | 0.0625 |
| 79043018 | .2500 | 00000835 | 0.1250 |
| 74052806 | 1.0000 | 78100502 | 0.0625 |
| 77051703 | 1.0000 | 79011101 | 0.1250 |
| 78031573 | .5000 | 00000226 | 0.1250 |
| 76090707 | .5000 | 72052308 | 0.1250 |
| 78120402 | 1.0000 | 76070106 | 0.1250 |
| 79082802 | 1.0000 | 76070103 | 0.5000 |
| 78071017 | .5000 | 76070101 | 1.0000 |
| 72012521 | 1.0000 | 76070104 | 1.0000 |
| 78040601 | 1.0000 | 76100703 | 0.1250 |
| 72052304 | 4.0000 | 76070102 | 1.0000 |
| 78071014 | 1.0000 | 77040102 | 0.0625 |
| 79021510 | .2500 | 80090204 | 0.1250 |
| 77072606 | 1.0000 | 77041308 | 0.0625 |
| 72052302 | 2.0000 | 76070110 | 1.0000 |
| 80011006 | .1250 | 78041902 | >64.0000 |
| 80090894 | 2.0000 | 77081817 | >64.0000 |
| 75100802 | .5000 | | |

TABLE 2 (continued)

| Organism | MIC | Organism | MIC |
|---|---|---|---|
| 77072603 | 1.0000 | 78060814 | 2.0000 |
| 79013132 | .5000 | 78121401 | 0.2500 |
| 77072718 | 2.0000 | 79110501A | 0.1250 |
| 80090865 | .1250 | 80090886 | 0.5000 |
| 75100801 | 1.0000 | 80090887 | 0.2500 |
| 80020808 | 4.0000 | 80090888 | 0.2500 |
| 80090880 | 2.0000 | 80090889 | 0.1250 |
| 80091805 | 2.0000 | 00027626 | >64.000 |
| *Serratia* | | *Streptococcus* | |
| 77080972 | .2500 | 0969/72 | 16.000 |
| 79121123 | .5000 | Z | 8.0000 |
| 75082819 | 4.0000 | 80030305B | 4.0000 |
| 80103103 | .2500 | 79101803B | 16.0000 |
| 75012721 | 1.0000 | 79112608 | 8.0000 |
| 74041505 | 1.0000 | Group D 79112502B | 16.0000 |
| 79030102 | 1.0000 | WOOD | 8.0000 |
| 79030606 | .5000 | 78091823A | 16.0000 |
| 75082815 | 1.0000 | 78091813A | 16.0000 |
| 79121114 | 4.0000 | 78040654A | 16.0000 |
| 77080914 | 4.0000 | 78091822D | 16.0000 |
| 77072705 | 1.0000 | ATCC29212 | |
| 76062203 | 1.0000 | *Streptococcus* | |
| 75032101 | 8.0000 | 80060504 | 0.0625 |
| 75041604 | 4.0000 | Sanders-4 | 0.2500 |
| 75042111 | 4.0000 | 488—78 | 0.1250 |
| 76081805 | 2.0000 | Viridans | |
| 74121201 | 1.0000 | Sanders 2 | 0.0313 |
| 79100302 | 1.0000 | BL 3848 | 0.1250 |
| 79082202 | 1.0000 | 918—78 | 0.1250 |
| 72012524 | 1.0000 | 1063—78 | 0.0625 |
| 79060504 | .5000 | 516—78 | 0.1250 |

| Organism | MIC | Organism | MIC |
|---|---|---|---|
| 76091606 | 2.0000 | *Streptococcus* | |
| 76032305 | 4.0000 | C Murc | 0.0625 |
| 78080424 | 2.0000 | C Thacker | <0.0313 |
| 75042113 | 8.0000 | G Parsons | 0.0625 |
| 77012512 | 2.0000 | A Hamer | 0.1250 |
| 80022917 | 8.0000 | A G—F | <0.0313 |
| 74011803 | 8.0000 | A Harper | <0.0313 |
| 80090809 | 16.0000 | B Lang | 0.1250 |
| 80090867 | 8.0000 | B Petruzzlia | 0.0625 |
| 80103122 | 1.0000 | B G—D | 0.1250 |
| *Proteus mirabilis* | | | |
| 78031559 | 1.0000 | | |
| 78091110 | 2.0000 | | |
| 79071204 | 1.0000 | | |
| 78080422 | 1.0000 | | |
| 79030912 | 0.5000 | | |
| 76072701 | 2.0000 | | |
| 77080944 | 4.0000 | | |
| 78031301 | 2.0000 | | |
| 79050223 | 0.5000 | | |
| 72012514 | 4.0000 | | |
| 79013103 | 4.0000 | | |
| 79013130 | 4.0000 | | |
| 79050218 | 1.0000 | | |
| 00012453 | 1.0000 | | |
| 72012515 | 0.5000 | | |
| 78022349 | 2.0000 | | |

**Claims for the Contracting States: BE CH DE FR GB IT LI NL SE**

1. A compound of the formula

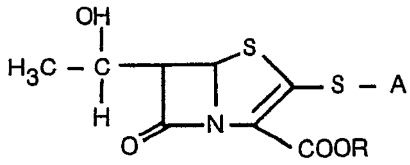

I

having the stereochemistry 5R,6S,8R or 5R,6R,8S, in which A represents a fluoroalkyl group having 2 to 4 carbon atoms, straight chain or branched chain, with 1 to 3 fluorine atoms on a single carbon atom which is separated from the 2—S atom by at least one carbon atom; and R is hydrogen, a pharmaceutically acceptable cation, or metabolisable ester group.

2. Sodium or potassium (5R,6S,8R)-6-(1-hydroxyethyl)-2-(2′-fluoroethylthio)-penem-3-carboxylate.

3. Sodium or potassium (5R,6S,8R)-6-(1-hydroxyethyl)-2-(3′-fluoro-propylthio)-penem-3-carboxylate.

4. Phthalidyl or pivaloyloxymethyl (5R,6S,8R)-6-(1-hydroxyethyl)-2-(2′-fluoroethylthio)-penem-3-carboxylate.

5. Phthalidyl or pivaloyloxymethyl (5R,6S,8R)-6-(1-hydroxyethyl)-2-(3′-fluoro-propylthio)-penem-3-carboxylate.

6. (5R,6S,8R)-6-(1-hydroxyethyl)-2-(2′-fluoroethylthio)-penem-3-carboxylic acid.

7. (5R,6S,8R)-6-(1-hydroxyethyl)-2-(3′-fluoro-propylthio)-penem-3-carboxylic acid.

8. A pharmaceutical composition comprising an antibacterially effective amount of a compound of any one of the preceding claims together with a non-toxic pharmaceutically acceptable carrier.

9. An oral dosage form comprising an antibacterially effective amount of a compound of any one of claims 1 to 7 in combination with a pharmaceutically acceptable carrier.

10. A process for the production of a compound of formula I as defined in Claim 1, characterized in that the compound is prepared by a process chosen from the following:

A. (for the production of compounds of formula I in which A is monofluoroalkyl), fluoridating a compound having the formula

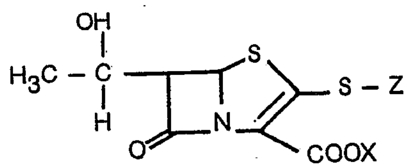

II

in which the stereochemistry is 5R,6S,8R or 5R,6R,8S, in which Z is an alkyl group having 2 to 4 carbon atoms, straight chain or branched chain, substituted by an atom or group Lg replaceable by fluorine under the conditions of the reaction, said atom or group Lg being separated from the 2—S atom by at least 2 carbon atoms, X is a carboxy protecting group, and the hydroxy group at the position 8 of the penem is protected if required;

B. alkylating a compound having the formula

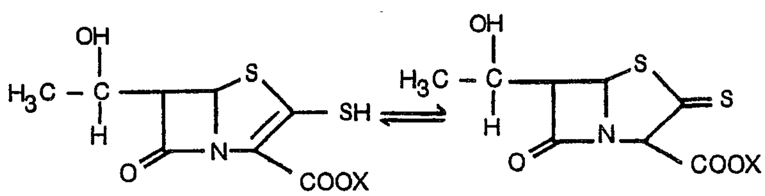

IIIa IIIb

in which the stereochemistry is 5R,6S,8R or 5R,6R,8S, X is as defined above, and the hydroxy group at position 8 of the penem is optionally protected, with a compound of the formula

G—Y IV

in which G is an alkyl group having 2 to 4 carbon atoms, straight chain or branched chain, substituted by 1 to 3 fluorine atoms on the same carbon atom which is separated from the atom or group Y by at least one carbon atom, and Y is an atom or group leaving under the conditions of the reaction;

C. Reacting a compound of the formula

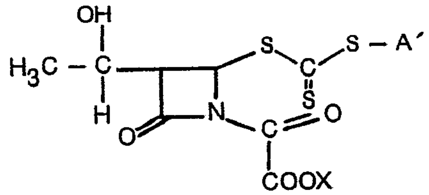

V

in which the stereochemistry is 3S,4R,5R, or 3R,4R,5S, A' is a fluoroalkyl group, straight or branched chain, with 1 to 3 fluorine atoms on the same carbon atom which is separated from the adjacent sulphur atom by at least one carbon atom, X is as defined in part A above and if necessary or desired the 5 position hydroxy group is protected;
with a trivalent organophosphorus compound;
followed by treatment of the reaction product from process A, B, or C, if necessary or desired, so as to remove any protecting groups, and isolation thereof as a free acid, or pharmaceutically acceptable salt or metabolisable ester.

**Claims for the Contracting State: AT**

1. A process for the production of a compound having the formula

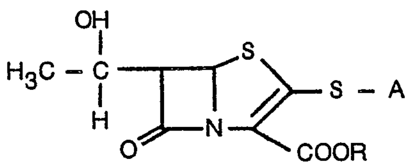

I

in which A represents a fluoroalkyl group, straight chain or branched chain, with 1 to 3 fluorine atoms on a single carbon atom which is separated from the 2—S atom by at least 2 carbon atoms, R is hydrogen a pharmaceutically acceptable cation or metabolisable ester group, and the stereochemistry is 5R,6S,8R or 5R,6R,8S, characterized in that the compound is prepared by a process chosen from the following:

A. (for producing a compound of formula I in which A is monofluoroalkyl), fluoridating a compound having the formula

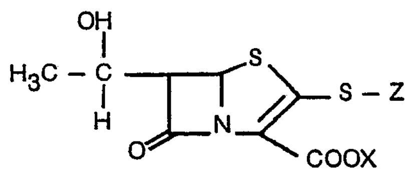

II

in which the stereochemistry is as defined for formula I, in which Z is an alkyl group having 2 to 4 carbon atoms, straight chain or branched chain, substituted by group Lg replaceable by fluorine under the conditions of the reaction, said group Lg being separated from the 2—S atom by at least 2 carbon atoms, X is a carboxy protecting group, and the hydroxy group at position 8 of the penem is protected if required,

B. alkylating a compound having the formula

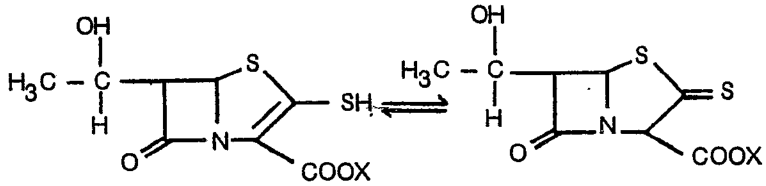

III

in which the stereochemistry is as defined for formula I, X is as defined in part A above, and the hydroxy group at position 8 of the penem is optionally protected, with a compound of the formula

G—Y IV

in which G is an alkyl group having 2 to 4 carbon atoms, straight chain or branched chain, substituted by 1 to 3 fluorine atoms on the same carbon atom which is separated from the group Y by at least one carbon atom, and Y is a group leaving under the conditions of the reaction,

C. Reacting a compound of the formula

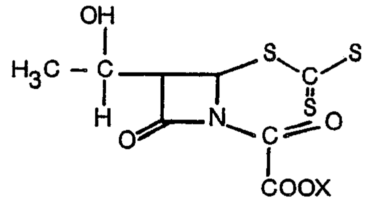

V

in which the stereochemistry is 3S,4R,5R, or 3R,4R,5S, A′ is a fluoroalkyl group, straight or branched chain, with 1 to 3 fluorine atoms on the same carbon atom which is separated from the adjacent sulphur atom by at least one carbon atom, X is as defined in part A above and if necessary or desired the 5 position hydroxy group is protected;

with a trivalent organophosphorus compound; followed by treatment of the reaction product from process A, B, or C, if necessary or desired, so as to remove any protecting groups, and isolation thereof as a free acid, or pharmaceutically acceptable salt or metabolisable ester.

2. A process according to Claim 1, in which the fluoridation is effected by reacting a compound of formula II in which Lg is hydroxy with diethylaminosulfur-trifluoride in a non-hydroxylic solvent at approximately neutral pH.

3. A process according to Claim 1 or 2, in which sodium or potassium (5R,6S,8R)-6-(1-hydroxyethyl)-2-(2′-fluoroethylthio)-penem-3-carboxylate is produced.

4. A process according to Claim 1 or 2, in which sodium or potassium (5R,6S,8R)-6-(1-hydroxyethyl)-2-(3′-fluoropropylthio)-penem-3-carboxylate is produced.

5. A process according to Claim 1 or 2, in which phthalidyl or pivaloyloxymethyl (5R,6S,8R)-6-(1-hydroxyethyl)-2-(2′-fluoroethylthio)-penem-3-carboxylate is produced.

6. A process according to Claim 1 or 2, in which phthalidyl or pivaloyloxymethyl (5R,6S,8R)-6-(1-hydroxyethyl)-2-(3′-fluoro-propylthio)-penem-3-carboxylate is produced.

7. A process according to Claim 1 or 2, in which (5R,6S,8R)-6-(1-hydroxyethyl)-2-(2′-fluoroethylthio)-penem-3-carboxylic acid is produced.

8. A process according to Claim 1 or 2, in which (5R,6S,8R)-6-(1-hydroxyethyl)-2-(3′-fluoro-propylthio)-penem-3-carboxylic acid is produced.

9. A process for preparing a pharmaceutical composition which comprises mixing a compound of formula I as defined in Claim 1 or as named in any one of Claims 3 to 8, with a pharmaceutically acceptable carrier or excipient.

10. A process according to Claim 9, in which the composition is produced as a pharmaceutical oral dosage form.

**Patentansprüche für die Vertragsstaaten: BE CH DE FR GB IT LI NL SE**

1. Verbindung der Formel

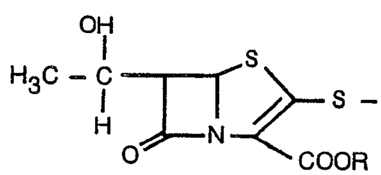

I

mit der Stereochemie 5R,6S,8R oder 5R,6R,8S, in welcher A eine geradkettige oder verzweigte Fluoralkylgruppe mit 2 bis 4 Kohlenstoffatomen und mit 1 bis 3 Fluoratomen an einem einzigen Kohlenstoffatom, das von dem 2-S-Atom durch mindestens ein Kohlenstoffatom getrennt ist, darstellt, und R Wasserstoff, ein pharmazeutisch annehmbares Kation oder eine metabolisierbare Estergruppe bedeutet.

2. Natrium- oder Kalium-(5R,6S,8R)-6-(1-hydroxyethyl)-2-(2′-fluorethylthio)-penem-3-carboxylat.

3. Natrium- oder Kalium-(5R,6S,8R)-6-(1-hydroxyethyl)-2-(3′-fluor-propylthio)-penem-3-carboxylat.

4. Phthalidyl- oder Pivaloyloxymethyl-(5R,6S,8R)-6-(1-hydroxyethyl)-2-(2′-fluorethylthio)-penem-3-carboxylat.

5. Phthalidyl- oder Pivaloyloxymethyl-(5R,6S,8R)-6-(1-hydroxyethyl)-2-(3′-fluor-propylthio)-penem-3-carboxylat.

6. (5R,6S,8R)-6-(1-Hydroxyethyl)-2-(2′-fluorethylthio)-penem-3-karbonsäure.

7. (5R,6S,8R)-(1-Hydroxyethyl)-2-(3′-fluor-propylthio)-penem-3-karbonsäure.

8. Pharmazeutische Zusammensetzung aus einer antibakteriell wirksamen Menge einer Verbindung nach einem der vorhergehenden Ansprüche und einem nicht-toxischen pharmazeutisch annehmbaren Träger.

9. Dosisform zur oralen Verabreichung aus einer antibakteriell wirksamen Menge einer Verbindung nach einem der Ansprüche 1 bis 7 und einem pharmazeutisch annehmbaren Träger.

10. Verfahren zur Herstellung einer Verbindung der in Anspruch 1 angegebenen Formel I, dadurch gekennzeichnet, daß die Verbindung nach einem aus den folgenden Verfahren ausgewählten Verfahren hergestellt wird:

A. (zur Herstellung von Verbindungen der Formel I, in welcher A für Monofluoralkyl steht) Fluorierung einer Verbindung der Formel

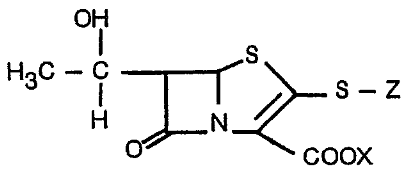

II

mit der Stereochemie 5R,6S,8R oder 5R,6R,8S, in welcher Z eine geradkettige oder verzweigte Alkylgruppe mit 2 bis 4 Kohlenstoffatomen bedeutet, die durch ein Atom oder eine Gruppe Lg substituiert ist, das bzw. die unter den Reaktionsbedingungen durch Fluor austauschbar ist, wobei das Atom oder die Gruppe Lg von dem 2-S-Atom durch mindestens 2 Kohlenstoffatome getrennt ist, X für eine Carboxyschutzgruppe steht und die Hydroxygruppe in Stellung 8 des Penems erforderlichenfalls geschützt ist;

B. Alkylierung einer Verbindung der Formel

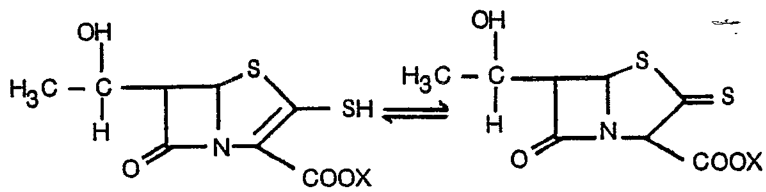

IIIa

IIIb

mit der Stereochemie 5R,6S,8R oder 5R,6R,8S, in welcher X obige Bedeutung hat und die Hydroxygruppe in Stellung 8 des Penems gegebenenfalls geschützt ist, mit einer Verbindung der Formel

G—Y IV

in welcher G eine geradkettige oder verzweigte Alkylgruppe mit 2 bis 4 Kohlenstoffatomen ist, die durch 1 bis 3 Fluoratome an demselben Kohlenstoffatom substituiert ist, welches von dem Atom oder der Gruppe Y durch mindestens ein Kohlenstoffatom getrennt ist, und Y für ein Atom oder eine Gruppe steht, das bzw. die sich unter den Reaktionsbedingungen abspaltet;

C. Umsetzung einer Verbindung der Formel

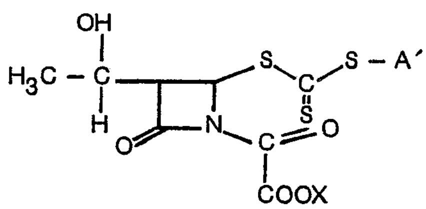

V

mit der Stereochemie 3S,4R,5R oder 3R,4R,5S, in welcher A' eine geradkettige oder verzweigte Fluoralkylgruppe mit 1 bis 3 Fluoratomen an demselben Kohlenstoffatom, das von dem benachbarten Schwefelatom durch mindestens ein Kohlenstoffatom getrennt ist, bedeutet, und X die in Absatz A angegebene Bedeutung hat und erforderlichenfalls oder gewünschtenfalls die Hydroxygruppe in 5-Stellung geschützt ist; mit einer dreiwertigen Organophosphorverbindung; erforderlichenfalls oder gewünschtenfalls anschließende Behandlung des Reaktionsproduktes aus dem Verfahren A, B oder C, um etwaige Schutzgruppen zu entfernen, und Isolierung desselben als freie Säure oder pharmazeutisch annehmbares Salz oder metabolisierbaren Ester.

**Patentansprüche für den Vertragsstaat: AT**

1. Verfahren zur Herstellung einer Verbindung mit der Formel

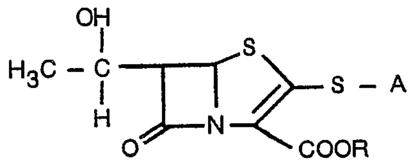

I

in welcher A eine geradkettige oder verzweigte Fluoralkylgruppe mit 1 bis 3 Fluoratomen an einem einzigen Kohlenstoffatom, das von dem 2-S-Atom durch mindestens 2 Kohlenstoffatome getrennt ist, und R Wasserstoff, ein pharmazeutisch annehmbares Kation oder eine metabolisierbare Estergruppe bedeutet, und mit der Stereochemie 5R,6S,8R oder 5R,6R,8S, dadurch gekennzeichnet, daß die Verbindung nach einem aus den folgengen Verfahren ausgewählten Verfahren hergestellt wird:

A. (zur Herstellung von Verbindungen der Formel I, in welcher A für Monofluoralkyl steht) Fluorierung einer Verbindung der Formel

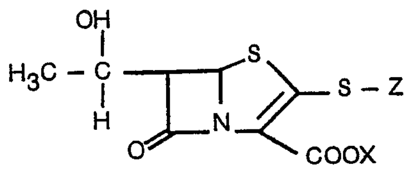

II

mit der für Formel I angegebenen Stereochemie, in welcher Z eine geradkettige oder verzweigte Alkylgruppe mit 2 bis 4 Kohlenstoffatomen bedeutet, die durch eine Gruppe Lg substituiert ist, die unter den Reaktionsbedingungen durch Fluor austauschbar ist, wobei die Gruppe Lg von dem 2-S-Atom durch mindestens 2 Kohlenstoffatome getrennt ist, X für eine Carboxyschutzgruppe steht und die Hydroxygruppe in Stellung 8 des Penems erforderlichenfalls geschützt ist,

B. Alkylierung einer Verbindung der Formel

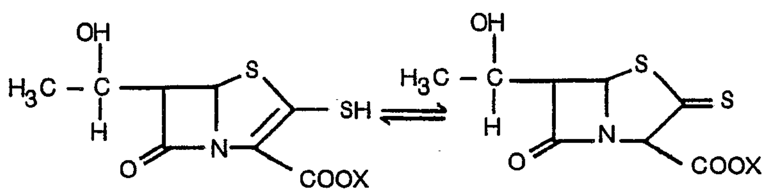

III

mit der für Formel I angegebenen Stereochemie, in welcher X die in Absatz A angegebene Bedeutung hat und die Hydroxygruppe in Stellung 8 des Penems gegebenenfalls geschützt ist, mit einer Verbindung der Formel

G—Y IV

in welcher G eine geradkettige oder verzweigte Alkylgruppe mit 2 bis 4 Kohlenstoffatomen ist, die durch 1 bis 3 Fluoratome an demselben Kohlenstoffatom substituiert ist, welches von der Gruppe Y durch mindestens ein Kohlenstoffatom getrennt ist, und Y für eine Gruppe steht, die sich unter den Reaktionsbedingungen abspaltet,

C. Umsetzung einer Verbindung der Formel

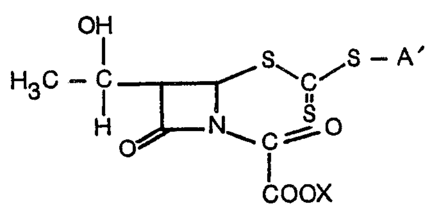

V

mit der Stereochemie 3S,4R,5R oder 3R,4R,5S, in welcher A′ eine geradkettige oder verzweigte Fluoralkylgruppe mit 1 bis 3 Fluoratomen an demselben Kohlenstoffatom, das von dem benachbarten Schwefelatom durch mindestens ein Kohlenstoffatom getrennt ist, bedeutet und X die in Absatz A

angegebene Bedeutung hat und erforderlichenfalls oder gewünschtenfalls die Hydroxygruppe in 5-Stellung geschützt ist; mit einer dreiwertigen Organophosphorverbindung;
erforderlichenfalls oder gewünschtenfalls anschließende Behandlung des Penem-Reaktionsproduktes aus dem Verfahren A, B oder C, um etwaige Schutzgruppen zu entfernen, und Isolierung desselben als freie Säure oder pharmazeutisch annehmbares Salz oder metabolisierbaren Ester.

2. Verfahren nach Anspruch 1A, in welchem die Fluorierung durch Umsetzung einer Verbindung der Formel II, in welcher Lg Hydroxy ist, mit Diethylaminoschwefeltrifluorid in einem hydroxylgruppenfreien Lösungsmittel bei etwa neutralem pH bewirkt wird.

3. Verfahren nach Anspruch 1 oder 2, in welchem Natrium- oder Kalium-(5R,6S,8R)-6-(1-hydroxyethyl)-2-(2′-fluorethylthio)-penem-3-carboxylat hergestellt wird.

4. Verfahren nach Anspruch 1 oder 2, in welchem Natrium- oder Kalium-(5R,6S,8R)-6-(1-hydroxyethyl)-2-(3′-fluorpropylthio)-penem-3-carboxylat hergestellt wird.

5. Verfahren nach Anspruch 1 oder 2, in welchem Phthalidyl- oder Pivaloyloxymethyl-(5R,6S,8R)-6-(1-hydroxyethyl)-2-(2′-fluorethylthio)-penem-3-carboxylat hergestellt wird.

6. Verfahren nach Anspruch 1 oder 2, in welchem Phthalidyl- oder Pivaloyloxymethyl-(5R,6S,8R)-6-(1-hydroxyethyl)-2-(3′-fluor-propylthio)-penem-3-carboxylat hergestellt wird.

7. Verfahren nach Anspruch 1 oder 2, in welchem (5R,6S,8R)-6-(1-hydroxyethyl)-2-(2′-fluorethylthio)-penem-3-karbonsäure hergestellt wird.

8. Verfahren nach Anspruch 1 oder 2, in welchem (5R,6S,8R)-6-(1-Hydroxyethyl)-2-(3′-fluor-propylthio)-penem-3-karbonsäure hergestellt wird.

9. Verfahren zur Herstellung einer pharmazeutischen Zusammensetzung, welches im Mischen einer Verbindung der in Anspruch 1 angegebenen Formel I oder wie sie in einem der Ansprüche 3 bis 8 genannt ist mit einem pharmazeutisch annehmbaren Träger oder Hilfsstoff besteht.

10. Verfahren nach Anspruch 9, in welchem die Zusammensetzung als eine pharmazeutische Dosisform zur oralen Verabreichung hergestellt wird.

**Revendications pour les Etats contractants: BE CH DE FR GB IT LI NL SE;**

1. Composé de formule

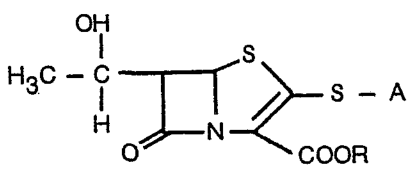

I

ayant la stéréochimie 5R,6S,8R ou 5R,6R,8S, où A représente un groupe fluoroalcoyle ayant 2 à 4 atomes de carbone, à chaîne droite ou à chaîne ramifiée, avec 1 à 3 atomes de fluor sur un seul atome de carbone qui est séparé de l'atome 2—S par au moins un atome de carbone; et R est hydrogène un cation acceptable en pharmacie ou un groupe ester métabolisable.

2. (5R,6S,8R)-6-(1-hydroxyéthyl)-2-(2′-fluoroéthylthio)-pénèm-3-carboxylate de sodium ou de potassium.

3. (5R,6S,8R)-6-(1-hydroxyéthyl)-2-(3′-fluoropropylthio)-pénèm-3-carboxylate de sodium ou de potassium.

4. (5R,6S,8R)-6-(1-hydroxyéthyl)-2-(2′-fluoroéthylthio)-pénèm-3-carboxylate de phtalidyle ou pivaloyloxyméthyle.

5. (5R,6S,8R)-6-(1-hydroxyéthyl)-2-(3′-fluoropropylthio)-pénèm-3-carboxylate de phtalidyle ou pivaloyloxyméthyle.

6. Acide (5R,6S,8R)-6-(1-hydroxyéthyl)-2-(2′-fluoroéthylthio)-pénèm-3-carboxylique.

7. Acide (5R,6S,8R)-6-(1-hydroxyéthyl)-2-(3′-fluoropropylthio)-pénèm-3-carboxylique.

8. Composition pharmaceutique comprenant une quantité bactéricidement efficace d'un composé selon l'une quelconque des revendications précédentes avec un véhicule non toxique acceptable en pharmacie.

9. Forme de dose orale comprenant une quantité bactéricidement efficace d'un composé selon l'une des revendications 1 à 7 en combinaison avec un véhicule acceptable en pharmacie.

10. Procédé pour la production d'un composé de formule I tel que défini à la revendication 1 caractérisé en ce que le composé est préparé par un procédé choisi parmi ceux qui suivent:

A. (pour la production de composés de formule I où A est monofluoroalcoyle), la fluoration d'un composé ayant la formule

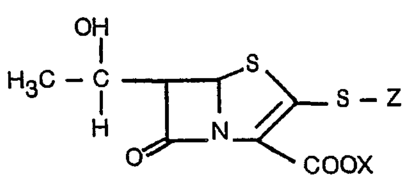

II

où la stéréochimie est 5R,6S,8R ou 5R,6R,8S, où Z est un groupe alcoyle ayant 2 à 4 atomes de carbone, à chaîne droite ou à chaîne ramifiée, substitué par un atome ou groupe Lg remplaçable par du fluor dans les conditions de la réaction, ledit atome ou groupe Lg étant séparé de l'atome 2—S par au moins 2 atomes de carbone, X est un groupe carboxy protecteur et le groupe hydroxy à la position 8 du pénème est protégé si cela est requis;

B. l'alcoylation d'un composé ayant pour formule

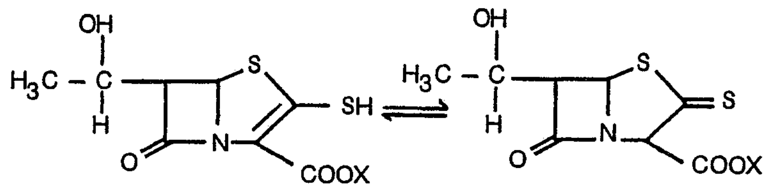

IIIa IIIb

où la stéréochimie est 5R,6S,8R ou 5R,6R,8S, X est tel que défini ci-dessus et le groupe hydroxy à la position 8 du pénème est éventuellement protégé, avec un composé de formule

G—Y IV

où G est un groupe alcoyle ayant 2 à 4 atomes de carbone, à chaîne droite ou à chaîne ramifiée, substitué par 1 à 3 atomes de fluor sur le même atome de carbone qui est séparé de l'atome ou groupe Y par au moins un atome de carbone et Y est un atome ou groupe partant dans les conditions de la réaction;

C. La réaction d'un composé de formule

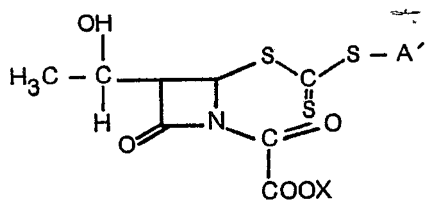

V

où la stéréochimie est 3S,4R,5R ou 3R,4R,5S, A' est un groupe fluoroalcoyle, à chaîne droite ou ramifiée, avec 1 à 3 atomes de fluor sur le même atome de carbone qui est séparé de l'atome de soufre adjacent par au moins un atome de carbone, X est tel que défini à la partie A ci-dessus et si nécessaire ou souhaitable, le groupe hydroxy à la position 5 est protégé;

avec un composé d'organophosphore trivalent;

avec ensuite traitement du produit réactionnel du procédé A, B ou C, si nécessaire ou souhaitable, afin de retirer tous groupes protecteurs et son isolement en tant qu'acide libre ou sel acceptable en pharmacie ou ester métabolisable.

**Revendications pour l'Etat contractant: AT**

1. Procédé pour la production d'un composé ayant pour formule

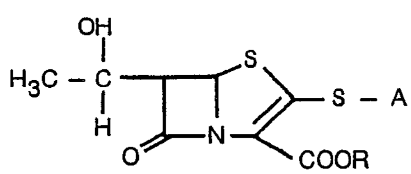

I

où A représente un groupe fluoroalcoyle, à chaîne droite ou à chaîne ramifiée, avec 1 à 3 atomes de fluor sur un seul atome de carbone qui est séparé de l'atome 2—S par au moins 2 atomes de carbone, R est hydrogène un cation acceptable en pharmacie ou groupe ester métabolisable et la stéréochimie est 5R,6S,8R ou 5R,6R,8S, caractérisé en ce que le composé est préparé par un procédé choisi parmi ceux qui suivant:

A. (Pour la production d'un composé de formule I où A est monofluoroalcoyle), la fluoration d'un composé ayant la formule

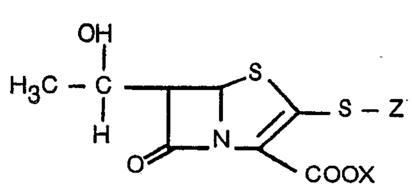

II

où la stéréochimie est telle que défini pour la formule 5, où Z est un groupe alcoyle ayant 2 à 4 atomes de carbone, à chaîne droite ou à chaîne ramifiée, substitué par un groupe Lg remplaçable par du fluor dans les conditions de la réaction, ledit groupe Lg étant séparé de l'atome 2—S par au moins 2 atomes de carbone, X est un groupe carboxy protecteur et le groupe hydroxy à la position 8 du pénème est protégé si cela est requis;

B. L'alcoylation d'un composé ayant pour formule

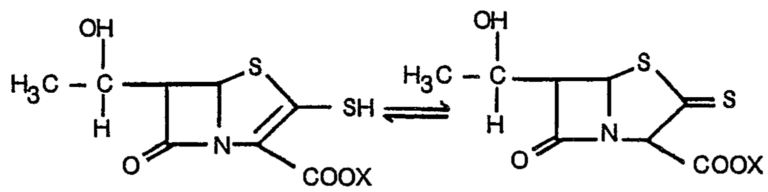

III

où la stéréochimie est telle que définie pour la formule 5, X est tel que défini en A ci-dessus et le groupe hydroxy à la position 8 du pénème est éventuellement protégé, avec un composé de formule

G—Y IV

où G est un groupe alcoyle ayant 2 à 4 atomes de carbone, à chaîne droite ou à chaîne ramifiée, substitué par 1 à 3 atomes de fluor sur le même atome de carbone qui est séparé du groupe Y par au moins un atome de carbone et Y est un groupe partant dans les conditions de la réaction;

C. La réaction d'un composé de formule

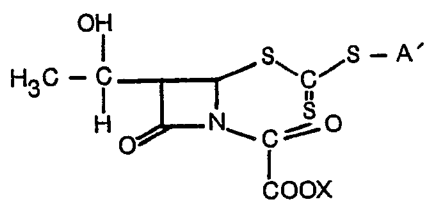

V

où la stéréochimie est 3S,4R,5R ou 3R,4R,5S, A' est un groupe fluoroalcoyle, à chaîne droite ou ramifiée, avec 1 à 3 atomes de fluor sur le même atome de carbone qui est séparé de l'atome de soufre adjacent par au moins un atome de carbone, X est tel que défini à la partie A ci-dessus et si nécessaire ou souhaitable, le groupe hydroxy à la position 5 est protégé;

avec un composé d'organophosphore trivalent;

avec ensuite traitement du produit réactionnel du procédé A, B ou C, si nécessaire ou souhaitable, afin de retirer tous groupes protecteurs et son isolement en tant qu'acide libre ou sel acceptable en pharmacie ou ester métabolisable.

2. Procédé selon la revendication 1A, où la fluoration est effectuée par réaction d'un composé de formule II où Lg est hydroxy avec du trifluorure de diéthylaminosoufre dans un solvant non hydroxyle à environ un pH neutre.

3. Procédé selon la revendication 1 ou 2 où on produit du (5R,6S,8R)-6-(1-hydroxyéthyl)-2-(2'-fluoroéthylthio)-pénèm-3-carboxylate de sodium ou potassium.

4. Procédé selon la revendication 1 ou 2 où l'on produit du (5R,6S,8R)-6-(1-hydroxyéthyl)-2-(3'-fluoropropylthio)-pénèm-3-carboxylate de sodium ou potassium.

5. Procédé selon la revendication 1 ou 2 où l'on produit du (5R,6S,8R)-6-(1-hydroxyéthyl)-2-(2'-fluoroéthylthio)-pénèm-3-carboxylate de phtalidyle ou pivaloyloxyméthyle.

6. Procédé selon la revendication 1 ou 2 où l'on produit du (5R,6S,8R)-6-(1-hydroxyéthyl)-2-(3'-fluoropropylthio)-pénèm-3-carboxylate de phtalidyle ou pivaloyloxyméthyle.

7. Procédé selon la revendication 1 ou 2 où l'on produit de l'acide (5R,6S,8R)-6-(1-hydroxyéthyl)-2-(2'-fluoroéthylthio)-pénèm-3-carboxylique.

8. Procédé selon la revendication 1 ou 2 où l'on produit de l'acide (5R,6S,8R)-6-(1-hydroxyéthyl)-2-(3'-fluoro-propylthio-pénèm-3-carboxylique.

9. Procédé de préparation d'une composition pharmaceutique qui consiste à mélanger un composé de formule I tel que défini à la revendication 1 ou tel que nommé selon l'une des revendications 3 à 8, avec un véhicule ou excipient acceptable en pharmacie.

10. Procédé selon la revendication 9, où la composition est produite sous la forme d'une dose orale pharmaceutique.